# EUROPÄISCHE PATENTSCHRIFT

(11) **EP 1 763 577 B1**
(45) Veröffentlichungstag und Bekanntmachung des Hinweises auf die Patenterteilung: **06.10.2010**
(21) Anmeldenummer: 05756002.1
(22) Anmeldetag: 04.06.2005
(51) Int. Cl.: C12N 9/04

(54) **ALKOHOLDEHYDROGENASE ZUR STEREOSELEKTIVEN GEWINNUNG VON HYDROXYVERBINDUNGEN**
ALCOHOL DEHYDROGENASE FOR THE STEREOSELECTIVE PRODUCTION OF HYDROXY COMPOUNDS
ALCOOL DESHYDROGENASE POUR LA PRODUCTION STEREOSELECTIVE DE COMPOSES HYDROXY

(30) Priorität: 11.06.2004 DE 102004029112
(43) Veröffentlichungstag der Anmeldung: 21.03.2007
(73) Patentinhaber: Codexis, Inc., Redwood City, CA 94063 (US)
(72) Erfinder: DAUSSMANN, Thomas, 40217 Düsseldorf (DE); HENNEMANN, Hans-Georg, 50181 Bedburg (DE)
(74) Vertreter: Leifert & Steffan
(86) Internationale Anmeldenummer: PCT/EP2005/006034
(87) Internationale Veröffentlichungsnummer: WO 2005/121326

(56) Entgegenhaltungen:
- WO-A-97/32012
- WO-A-03/091423
- DE-A1- 4 209 022
- US-A- 5 908 924
- BURDETTE DOUGLAS S ET AL: "Cloning and expression of the gene encoding the Thermoanaerobacter ethanolicus 39E secondary-alcohol dehydrogenase and biochemical characterization of the enzyme" BIOCHEMICAL JOURNAL, Bd. 316, Nr. 1, 1996, Seiten 115-122, XP002355639 ISSN: 0264-6021
- DATABASE EMBL-SVA [Online] issued: 18-Feb 1997; 8. Januar 1997 (1997-01-08), BURSTEIN Y: "T.brockii TBAD gene for alcohol dehydrogenase" XP002355678 Database accession no. X64841
- DATABASE EMBL [Online] 1. März 2004 (2004-03-01), BAO Q ET AL: "Threonine dehydrogenase and related Zn-dependent dehydrogenases" XP002355679 Database accession no. Q8RBW3 -& BAO QIYU ET AL: "A complete sequence of the T. tengcongensis genome." GENOME RESEARCH. MAY 2002, Bd. 12, Nr. 5, Mai 2002 (2002-05), Seiten 689-700, XP002355673 ISSN: 1088-9051
- ECKSTEIN M ET AL: "Recent developments in NAD(P)H regeneration for enzymatic reductions in one- and two-phase system" BIOCATALYSIS AND BIOTRANSFORMATION 2004 UNITED KINGDOM, Bd. 22, Nr. 2, 2004, Seiten 89-96, XP008056129 ISSN: 1024-2422

## Beschreibung

Die vorliegende Erfindung betrifft ein DNA-Molekül, das für eine NADP-abhängige Alkoholdehydrogenase kodiert, einen Vektor, der mindestens eine Kopie der DNA-Sequenz enthält, und prokaryontische oder eukaryontische Wirtszellen, die mit der DNA-Sequenz transformiert oder transfiziert sind. Die Erfindung betrifft auch die NADP-abhängige Alkoholdehydrogenase, ein Verfahren zur Herstellung und die Verwendung der Alkoholdehydrogenase sowie ein Verfahren zur stereoselektiven Herstellung von sekundären Alkoholen.

Alkoholdehydrogenasen sind eine gut bekannte Klasse von Enzymen, mit denen Ketoverbindungen enzymatisch zu Alkoholen reduziert werden können.

Die Gewinnung optisch aktiver organischer Verbindungen, z. B. von Alkoholen und Aminen, auf biokatalytischem Weg gewinnt zunehmend an Bedeutung. Als ein Weg zur großtechnischen Synthese dieser Verbindungen ist der gekoppelte Einsatz zweier Dehydrogenasen unter Cofaktorregenerierung aus der DE 197 53 350 A1 bekannt.

Die In-sito-Regeneration von NADPH mit einer NADP-abhängigen Glukose-Dehydrogenase, Glukose-6-Phosphatdehydrogenase oder anderen NADP-abhängigen Oxidoreduktasen bietet sich hier an (vgl. Y. Yasohara, N. Kizaki, J. Hasegawa, M. Wada, M. Kataoka und S. Shimizu, Tetrahedron: Asymmetry 12 (2001) 1713-1718).

Alkoholdehydrogenasen (ADHs) sind in diesem Zusammenhang interessant, erlauben sie doch in einem gleichgelagerten gekoppelten enzymatischen System u.a. die Herstellung von enantiomer angereicherten Alkoholen ausgehend von Ketonen durch enantioselektive Reduktion, oder aus racemischen Alkoholen durch kinetische Racematspaltung (DE 100 37 101; als aktuelle, umfassende Übersicht zum Stand der Technik siehe: W. Hummel, Adv. Biochem. Engineering/Biotechnology 1997, 58, 145-184).

Ebenfalls viel genutzt ist die substratgekoppelte Cofaktorregenerierung, die ohne eine zweite Dehydrogenase auskommt. Der Cofaktor wird hier durch die zum gewünschten Umsatz erforderliche Alkoholdehydrogenase unter Verwendung eines im Überschuß zugesetzten Zweitsubstrates (beliebiger Alkohol wie z.B. Isopropanol und Ethanol) in umgekehrter Syntheserichtung regeneriert (vgl. W. Stampfer, B. Kosjek, C. Moitzi, W. Kroutil and K. Faber Angew Chem Int Ed Engl. 2002 Mar 15;41(6):1014-7). ADHs werden in die Klasse E.C. 1.1.1.1 bzw. E.C.1.1.1.2 eingeteilt und gehören damit zu den sogenannten Oxidoreduktasen. Sie finden sich in einer Reihe von Organismen (Enzyme Catalysis in Organic Synthesis, Ed.: K. Drauz and H. Waldmann, 1995, VCH, Vol. II. 595ff).
Interessant sind "Breitbandenzyme", die stereoselektiv ein breites Spektrum an Substraten umsetzen.

Kommerziell erhältlich für die präparative Anwendung im Labormaßstab sind z.B. die ADH aus Hefe (YADH), aus Pferdeleber (HLADH) und aus *Thermoanaerobacter brokii* bzw. *Thermoanaerobium brockii,* die zur Herstellung von Alkoholen verwendet werden. Daneben gibt es eine Reihe weiterer ADHs zum käuflichen Erwerb, die aber eher, wie der Name schon sagt, spezielle Substrate umsetzen, wie z.B. einige Steroid-Dehydrogenasen, die bevorzugt Alkoholgruppen an Steroid-Strukturen umsetzen oder Glycerol-Dehydrogenasen, die Glycerin umsetzen oder letztlich auch Zucker umsetzende Enzyme wie die schon genannte Glucose-Dehydrogenase.

Die meisten der bislang literatur-bekannten ADHs sind "S-spezifisch" (wobei sich die Bezeichnung S und R aus formellen Gründen der Nomenklatur manchmal auch umkehren kann). Dagegen sollen die ADHs aus den *Lactobacillus*-Stämmen R-spezifisch sein (siehe C.W. Bradshaw, W. Hummel, C.-H. Wong, J. Org. Chem. 1992, 57, 1532.) sowie eine weitere literaturbekannte aus *Pseudomonas* (P. Hildebrandt, T. Riermeier, J. Altenbuchner, U. T. Bornscheuer, Tetrahedron: Asymmetry 2001, 12, 1207.), die von der Arbeitsgruppe Altenbuchner und Bornscheuer kürzlich beschrieben wurde. Der Arbeitskreis um Keinan und Lamed berichtete zudem über eine ADH aus *Thermoanaerobium brockii* (E. Keinan, E. K. Hafeli, K. K. Seth, R. Lamed, J. Am. Chem. Soc. 1986, 108, 162.) die für kleine Substrate eine(R)-Spezifität zeigt, für größere Substrate dagegen (S)-spezifisch ist.

Die in dieser Erfindung beschriebene ADH T ist (S)-selektiv. Bei den (S)-spezifischen Alkoholdehydrogenasen sind zwar eine Reihe von Vertretern bekannt, wobei deren technische Eignung zumeist sehr beschränkt ist. Dies dokumentiert nicht zuletzt die kaum vorhandenen industriellen Nutzungsverfahren mit solchen Enzymen im Gegensatz zu der Vielzahl an bekannten ADHs. Die (S)-ADH aus Hefe ist zwar ein preiswertes NADH-abhängiges Enzym, setzt aber bevorzugt primäre Alkohole um, so daß sie für die Herstellung chiraler Alkohole wenig Bedeutung hat.

Die NADH-abhängige (S)-ADH aus Pferdeleber (HLADH) stellt zweifelsohne die bislang gerade im akademischen Bereich am häufigsten angewendete Alkoholdehydrogenase dar, wie die Vielzahl an Publikationen mit diesem Enzym verdeutlicht (siehe z.B. Übersicht in: K. Faber, Biotransformations in Organic Chemistry, 4. Ausgabe, Springer-Verlag, 2000, p. 184f). Leider kommt dieses Enzym für eine technische Nutzung aufgrund der mangelnden Verfügbarkeit kaum in Frage. (S)-ADH aus Pferdeleber ist sehr teuer (1U ca. 0,5 Euro), da sie bisher nicht rekombinant verfügbar ist.

Das Substratspektrum umfaßt zudem bevorzugt cyclische Ketone, sie setzt keine Ketone mit aromatischen Seitenketten um (Typ Acetophenon). Diese Substanzklasse der aromatischen Ketone besitzt aber gerade aus industrieller Sich hohe Bedeutung aufgrund deren Vielzahl an Anwendungen als Schlüsselintermediate im pharmazeutischen Bereich (für ausgewählte Beispiele, siehe: a) R.A. Holt, S. R. Rigby (Zeneca Limited), US 5580764, 1996; b) T.J. Blacklock, P. Sohar, J.W. Butcher, T. Lamanec, E.J.J. Grabowski, J. Org. Chem. 1993, 58, 1672-1679; c) R.A. Holt, Chimica Oggi - Chemistry Today 1996, 9, 17-20; d) F. Bracher, T. Litz, Arch. Pharm. 1994, 327, 591-593; e) S.Y. Sit, R.A. Parker, I. Motoc, W. Han, N. Balasubramanian, J. Med. Chem. 1990, 33, 2982-2999; f) A. Zaks, D.R. Dodds, Drug Dicovery Today 1997, 2, 513-530).

Die NADP-abhängige ADH (TBADH) aus *Thermoanaerobacter brockii* bzw. *Thermoanaerobium brockii* ist rekombinant verfügbar. Der Preis für NADP ist etwa 3-4 mal so hoch wie der für NAD. Die Kosten des Cofaktors bei Verwendung eines geeigneten Regenerationssystems sind dennoch akzeptabel. Das Substratspektrum der TBADH beschränkt sich jedoch auf aliphatische Ketone. Es werden beispielsweise keine Ketone mit aromatischen Seitenketten (Typ Acetophenon) umgesetzt.

Eine weitere gut verfügbare (S)-selektive ADH stellt das Enzym aus *Rhodococcus erythropolis* dar (DE 42 09 022.9, WO03/091423). Es wird hier ein breites Substratspektrum akzeptiert. Nachteil dieser ADH für eine technische Anwendung ist allerdings die Notwendigkeit einer enzymgekoppelten Cofaktorregenerierung. Ein preiswertes Zweitsubstrat wie beispielsweise Isopropanol kann nicht eingesetzt werden.

Dagegen kann bei der (S)-ADH aus Rhodococcos *ruber* (W. Stampfer, B. Kosjek, C. Moitzi, W. Kroutil and K. Faber, Angew Chem Int Ed Engl. 2002 Mar 15;41(6):1014-7) erfolgreich der substratgekoppelte Weg zur Cofaktorregenerierung beschritten wegen. Hier ist der wesentliche Nachteil in einer technischen Umsetzung in der limitierten Verfügbarkeit des Enzyms zu sehen, bisher kann das Enzym nur aus dem Wildtyp hergestellt werden, eine Klonierung und Überexpression war bisher nicht erfolgreich.

Offensichtlich besteht daher noch immer Bedarf an der Bereitstellung industriell interessanter ADHs ohne die oben genannten Nachteile.

Es ist Aufgabe der vorliegende Erfindung, eine neue stereoselektive Alkoholdehydrogenase und die für ihre rekombinante Herstellung notwendige DNA anzugeben.

Diese Aufgabe wird gelöst durch ein DNA-Molekül gemäß Anspruch 1 und eine Alkoholdehydrogenase gemäß Anspruch 5 und Anspruch 6.

Die erfindungsgemäße DNA stammt aus *Thermoanaerobacter species.*

Ausgehend von der Sequenz kann die DNA in bekannter Weise synthetisiert werden. Besonders geeignet sind hierzu automatisierte Festphasenmethoden, bei denen aktivierte Monomere aufeinanderfolgend an eine wachsende Kette addiert werden, die an eine unlösliche Matrix gebunden ist. Reaktive Gruppen, die während der Synthese nicht reagieren sollen, werden durch Schutzgruppen blockiert. Nach der vollständigen Synthese der DNA-Kette werden die Schutzgruppen abgespalten und der DNA-Strang von der Matrix abgelöst.

DNA-Moleküle mit einer vorgegebenen Sequenz sind auch kommerziell erhältlich. Sie werden von Firmen hergestellt, die sich auf die Herstellung beliebiger Sequenzen spezialisiert haben. Es ist also nicht mehr erforderlich, den Ausgangsorganismus zur Verfügung zu haben, um zu einem DNA-Molekül zu gelangen.

Der im Anspruch 1 verwendete Begriff "stringente Bedingungen" beschreibt Bedingungen für die Hydrolisierung und das Waschen der DNA. Stringente Bedingungen sind dem Fachmann bekannt und können in der Literatur gefunden werden (z. B. Current Protocols in Molecular Biology, John Wiley & Sons, N.Y., 1989, 6.3.1-6.3.6). In dieser Literaturstelle werden wässrige und nichtwässrige Verfahren beschrieben. Bevorzugte Beispiele stringenter Hybridisierungsbedingungen werden auch in dem US-Patent US-A-6 740 514 beschrieben.

Die erfindungsgemäße Alkoholdehydrogenase ist thermostabil und wird im Folgenden auch als ADH T bezeichnet. Das beschriebene Enzym setzt (S)-selektiv sowohl aliphatische Alkohole, Ketone und Diketone, als auch solche mit aromatischen Seitenketten (Typ Acetophenon) um.

Seine Thermostabilität ermöglicht Umsetzungen in einem weiten Temperaturbereich zwischen 30° und 70°C. Die Reaktionstemperatur kann durch die Verwendung einer thermostabilen Glucose-Dehydrogenase quasi ohne Rücksicht auf die Enzyme gewählt werden. Durch die Lösungsmitteltoleranz der ADH T ist die Cofaktorregenerierung jedoch auch substratgekoppelt, z.B. durch den Einsatz von Isopropanol, möglich.

Die Verwendung der erfindungsgemäßen Alkoholdehydrogenase wird schematisch in der folgenden Abbildung 1 gezeigt. 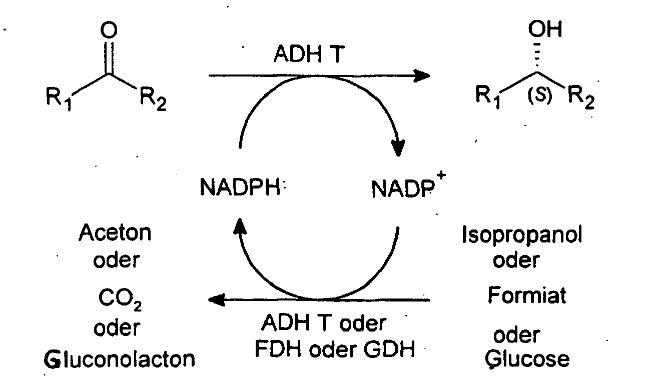 Abb. 1: Allgemeines Schema zum Einsatz der Alkoholdehydrogenase unter Verwendung einer Cofaktorregenerierung, dabei hat R₂ die höhere Raumerfüllung gegenüber R₁ (prostereogenen Seiten), der Angriff des prochiralen Ketons erfolgt von der Si-Seite

Aus dem US-Patent US 5 908 924 A1 oder WO 97/32012 ist eine Alkoholdehydrogenase aus *Thermoanaerobacter ethanolicus 39E* bekannt, deren DNA-Sequenz und Aminosäuresequenz eine gewisse Homologie zu dem erfindungsgemäßen DNA-Molekül und der erfindungsgemäßen ADH T aufweisen. Die in dem genannten US-Patent beschriebenen und beanspruchten DNA-Moleküle sind nicht Gegenstand der vorliegenden Erfindung, und die erfindungsgemäße Alkoholdehydrogenase ADH T weist gegenüber der Alkoholdehydrogenase aus *Thermoanaerobacter ethanolicus* 39E erhebliche nicht vorhersehbare Vorteile auf, wie im Folgenden durch Vergleichsversuche gezeigt wird.

Die vorliegende Erfindung betrifft auch einen Vektor, der mindestens eine Kopie der DNA-Sequenz nach Anspruch 1 enthält sowie eine prokaryontische oder eukaryontische Wirtszelle, die mit einer DNA-Sequenz nach Anspruch 1 in einer Weise transformiert oder transfiziert ist, die es der Wirtszelle erlaubt die genannte Alkoholdehydrogenase zu exprimieren. Der Wirt ist vorteilhaft ausgewählt aus der Gruppe bestehend aus *Escherichia coli, Saccharomyces cerevisiae, Pichia pastoris und Bacillus subtilis.*

Grundsätzlich können die Fermentation und die Expression zur Gewinnung der erfindungsgemäßen Alkoholdehydrogenase ADH T unter den dem Fachmann bekannten Bedingungen durchgeführt werden, wie sie z. B. von M.I. Viitanen, A. Vasala, P. Neubauer und T. Alatossava in Microbial Cell Factories 2003, 2:2 beschrieben sind. Dieses Vorgehen führt jedoch nur zu schwachen Aktivitätsausbeuten von ca. 200 U/g Zellfeuchtgewicht. Überraschender weise wurde gefunden, dass die Aktivitätsausbeute erheblich gesteigert werden kann, wenn dem Kulturmedium Zinkionen zugesetzt werden. So wurde durch den Zusatz von 1 mM Zinksulfat die Aktivitätsausbeute verzehnfacht. Die Erfindung betrifft daher auch ein Verfahren zur Herstellung der erfindungsgemäßen Alkoholdehydrogenase, bei dem prokaryontische oder eukaryontische Wirtszellen, die mit einer DNA-Sequenz nach Anspruch 1 derart transformiert oder transfiziert worden sind, dass es den Wirtszellen ermöglicht ist, die Alkoholdehydrogenase zu exprimieren, in einem Kulturmedium, das Zinkionen in einer Konzentration von 0,1 bis 5 mM enthält, kultiviert werden und die Alkoholdehydrogenase als Expressionsprodukt der DNA-Sequenz isoliert wird.

Die Abhängigkeit der Aktivitätsausbeute von der Zinkkonzentration ist in der folgenden Tabelle 1 dargestellt.

**Tabelle 1: Untersuchung verschiedener Zinkkonzentrationen in Hochzelldichtefermentation bei heterologer Expression der ADH T in E. coli. Induziert wurde bei OD₆₀₀ 30 mit 0.4 mM IPTG für 14 Stunden bei 30°C. Die Aktivität ist pro Gramm Zellfeuchtgewicht nach der Induktionsperiode angegeben.**

| Zinkkonzentration | 0,0 mM | 0,5 mM | 1,0 mM | 2,0 mM | 3,0 mM |
|---|---|---|---|---|---|
| Aktivität | 200 U/g | 2900 U/g | 2000 U/g | 1113 U/g | 1118 U/g |

Trotz ihrer Thermostabilität ist die isolierte ADH T unter Standardbedingungen (50 mM Tris-Cl, pH 7.0) nicht lange lagerfähig. Die Halbwertszeit beträgt nur ca. 4 Tage. Durch Zusatz von z.B. NaCl kann die Halbwertszeit auf mehr als 40 Tage gesteigert werden (Tabelle 2).

**Tabelle 2: Darstellung der Aktivität der Isolierten ADH T unter verschiedenen Lagerbedingungen bei 4°C**

| Konzentration NaCl | 0,0 M | 0,2 M | 0,5 M | 1,0 M |
|---|---|---|---|---|
| Aktivität Start | 105 U/mL | 105 U/mL | 105 U/mL | 105 U/mL |
| Aktivität Tag 5 | 24 U/mL | 101 U/mL | 113 U/mL | 115 U/mL |
| Aktivität Tag 10 | 6 U/mL | 7 U/mL | 86 U/mL | 119 U/mL |
| Aktivität Tag 20 | 0 U/mL | 0 U/mL | 0 U/mL | n.b. |
| Aktivität Tag 40 | 0 U/mL | 0 U/mL | 0 U/mL | 78 U/mL |

Der Zusatz von Zinkionen zum präparierten Enzym führt jedoch schnell zu dessen Inaktivierung.

Eine Lagerung bei -20°C zeigte hohe Stabilität über mindestens 3 Monate bei Zusatz von Glycerin, insbesondere in einer Konzentration von 50%.

Ebenfalls als Stabilisator der Enzympräparation im Sinne dieser Anmeldung geeignet sind Glykol, Polyethylenglykol, sowie Zucker wie z.B. Trehalose oder Saccharose und Kombinationen dieser Stoffe für sich oder in Verbindung mit einem Stoff zur geeigneten Erhöhung der Ionenstärke.

### Weitere Charakterisierung der rekombinanten ADH T:

Das pH-Optimum wurde mit pH 7.0 festgestellt (Tabelle 3 und 4).

**Tabelle 3: Darstellung der Aktivität der isolierten ADH T bei verschiedenen pH-Werten.**

| | | | | | |
|---|---|---|---|---|---|
| pH-Wert | 5.0 | 6.0 | 7.0 | 8.0 | 9.0 |
| gemessene Aktivität | 53 U/mL | 134 U/mL | 192 U/mL | 146 U/mL | 13 U/mL |

**Tabelle 4: Die Aktivität bei pH-Werten näher um pH 7,0 wurde noch einmal gesondert untersucht.**

| | | | |
|---|---|---|---|
| pH-Wert | 6,5 | 7,0 | 7,5 |
| relative Aktivität | 66 % | 100% | 74 % |

Auch die Thermostabilität ist sehr gut. Die Aktivität der ADH T betrug selbst nach 12 h bei 50°C noch 75% des Ausgangswertes.

Im hier beschriebenen aufgereinigten Zustand setzt sie aliphatische und aromatische Ketone, Aldehyde, 2- bzw. 3-Ketoester und Diketone um. Weitere Daten zum Substratspektrum sind in Tabelle 5 gezeigt.

**Tabelle 5: Relative Aktivitäten der aufgereinigten ADH T bei der Reduktion verschiedener Substrate (10 mM). Die Aktivität mit Aceton wurde gleich 100% gesetzt.**

| Substrat | Relative Aktivität |
|---|---|
| Aceton | 100 % |
| 2-Butanon | 72 % |
| 2-Pentanon | 31 % |
| 2-Hexanon | 30 % |
| 2-Oktanon | 9 % |
| Monochloraceton | 71 % |
| Acetaldehyd | 101 % |
| 2-Methylbutyraldehyd | 52 % |
| Diacetyl , | 154 % |
| 2,4-Pentandion | 77 % |
| 2,5-Hexandion | 74 % |
| Ethylacetoacetat | 22 % |
| Ethyl 4-Chloracetoacetat | 45 % |
| Acetophenon | 28 % |
| p-Chloracetophenon | 17 % |
| 2-Acetylpyridin | 36 % |
| 3-Butyn-2-on | 76 % |
| 1,2-Cyclohexandion | 5 % |
| Pyruvat | 2 % |
| 2-Oxobuttersäure | 28 % |
| 4-Chlor-2-butanon | 66 % |
| 4-Hydroxy-2-butanon | 13 % |
| 3-Oktanon | 26 % |
| Phenacylchlorid | 25 % |

Interessant ist der Vergleich der Aktivität der ADH T mit der der sekundären Alkoholdehydrogenase aus *Thermoanaerobacter ethanolicus **39**E* (US-A-5,908,924) im Hinblick auf die Umsetzung von Diketonen (Tabelle 6).

Die korrespondierenden Diole spielen eine große Rolle bei der Synthese chiraler Liganden chemischer Homogenkatalysatoren und müssen deshalb von höchster Enantiomeren- und Diastereomerenreinheit sein.

**Tabelle 6: Darstellung der relativen Aktivität der ADH T mit verschiedenen Diketonen.**

| Enzym | Standardsubstrat | 2,4-Pentandion | | 2,5-Hexandion | |
|---|---|---|---|---|---|
| | | rel. Aktivität | spez. Aktivität | rel. Aktivität | spez. Aktivität |
| ADH T 1,5 mg/mL | Aceton (10 mM) 106 U/mL | 77 % | 55,4 U/mg | 74 % | 52,7 U/mg |
| ADH-TE 2,45 mg/mL | Aceton (10 mM) 75 U/mL | 34 % | 10,3 U/mg | 12 % | 3,8 U/mg |

Die Umsetzung des 2,4-Pentandions mit ADH T eignet sich sowohl für die Herstellung des (S)-4-Hydroxy-2-pentanons, als auch des (S,S)-2,4-Pentandiols.

Die Reduktion kann beide Produkte in sehr hoher enantiomerer und diastereomerer Reinheit liefern (Tabelle 7).

**Tabelle 7: Gezeigt sind die gefundenen Substrat- und Produktmengenanteile nach 48 h Inkubation bei 30°C in 50 mM Tris/HCl pH 7,0. Zur Erzeugung des (S)-4-Hydroxy-2-pentanons wurden 5 U/mL Enzym mit durch Isopropanol (10%) substratgekoppelter Cofaktorregenerierung eingesetzt, zur Erzeugung des (S,S)-2,4-Pentandiols 10 U/mL mit durch Glucosedehydrogenase enzymgekoppelter Cofaktorregenerierung. Der Cofactor ist in beiden Fällen NADP.**

| | 2,4-Pentandion (nicht umgesetzt) | (R)-4-Hydroxy-2-pentanon | (S)-4-Hydroxy-2-pentanon | *meso*-2,4-Pentandiol | (S,S)-2,4-Pentandiol |
|---|---|---|---|---|---|
| ADH T substratgekoppelt | - | - | 82,6 % | - | 17,4 % |
| ADH T enzymgekoppelt | - | - | 14,6 % | - | 85,4 % |
| ADH-TE substratgekoppelt | 84,8 % | - | 0,6 % | 14,6 % | - |
| ADH-TE enzymgekoppelt | - | 7,1 % | 17,3 % | 15,7 % | 59,9 % |

Hier zeigt sich, dass die ADH T nicht nur hohe Umsätze erreicht, sondern im Gegensatz zur ADH-TE für die Reduktion des 2,4-Pentandions eine weit bessere Enantio- und Diastereoselektivität besitzt.

2,5-Hexandion wird von der ADH T ebenfalls mit sehr guten Werten für Umsatz und Enantio- bzw. Diastereoselektivität bis zum Diol reduziert (Tabelle 8).

**Tabelle 8: Gezeigt sind die Werte für Umsatz nach 48 h und ee/de für das (S,S)-Enantiomer bei gleich eingesetzten Enzymmengen wie in Tabelle 6.**

| | 2,5-Hexandion | |
|---|---|---|
| | Umsatz zum Diol | ee/de |
| ADH T substratgekoppelt | 10,6 % | 100 % |
| ADH T enzymgekoppelt | 82,4 % | 100 % |
| ADH-TE substratgekoppelt | 0,3 % | nicht bestimmbar |
| ADH-TE enzymgekoppelt | 3,3 % | nicht bestimmbar |

Der ee/de-Wert von 100% bedeutet absolute Enantiomeren- und Diastereomerenreinheit der dargestellten Diole. Mit Hilfe der hier beschriebenen Erfindung läßt sich die ADH T erstmals in ausreichender Menge gewinnen, um enantiomerenreine Diole im Kilogramm-Maßstab herzustellen.

Die durch die Anmeldung beschriebene Verbesserung der Stabilität des isolierten Enzyms steigert die Produktausbeute an chiralen Alkoholen in einer Biotransformation durch ADH T bezogen auf die eingesetzte Enzymmenge deutlich.

Tatsächlich läßt sich zeigen, daß die Enantioselektivität auch bei der Reduktion kritischer Ketone wie 2-Pentanon oder 2-Butanon durch substratgekoppelte Cofaktorregenerierung mit Isopropanol weit besser ist als bei der Verwendung der enzymgekoppelten Cofaktorregenerierung.

Dabei hängt die erreichbare enantiomere Reinheit des Produktes von der Menge des eingesetzten Isopropanols ab (Tabelle 9).

**Tabelle 9: Gezeigt sind die erreichten Umsätze und Enantiomerenüberschüsse (ee) für eine Umsetzung von 200 mM 2-Pentanon mit 3 U/mL ADH T, 0,05 mM NADP in 100 mM Tris-Cl pH 7.0 mit verschiedenen Isopropanolkonzentrationen zur substratgekoppelten Cofaktorregenerierung.**

| | 20 % Isopropanol | | 50 % Isopropanol | |
|---|---|---|---|---|
| | Umsatz | ee | Umsatz | ee |
| 24 Stunden | 85,3 % | 98,9 % | 93,8 % | 99,9 % |
| 48 Stunden | 86,5 % | 98,3 % | 94,2 % | 99,9 % |

### Aktivitätsmessung

Die Aktivitäten wurden durch photometrische Messung der Abnahme des Cofaktors bestimmt. Messansatz: 50 mM Tris-Cl, pH 7,0 mit 10 mM Substrat, 0,2 mM NADPH. Der Meßansatz für ADH-TE enthielt außerdem noch 1 mM MgCl₂.

Die Abnahme des NADPH wurde nach Zugabe des Enzyms bei 340 nm für eine Minute verfolgt. Es wurde soviel Enzym zugesetzt, dass die Aktivität zwischen 0,5 und 1,5 U/mL lag und das Messvolumen 1 mL betrug.

1 U entspricht der Enzymmenge, die 1 µmol Substrat pro Minute umsetzt.

### Klonierung der Alkohol-Dehydrogenase aus Thermoanaerobacter species

Zur Klonierung der Alkoholdehydrogenase aus *Thermoanaerobacter species* wurde angenommen, daß die entsprechende Gen-Sequenz der aus den verwandten Organismen *Th. ethanolicus, Th. brockii und Th. tengcongensis* ähnlich genug ist, um aus deren Sequenzen ein Primer-Paar abzuleiten.

Die abgeleiteten Primersequenzen lauten:
N: CAT ATG AAA GGT TTT GCA ATG CTC AGT ATC GG
C: CTC GAG TTA TGC TAA TAT TAC AAC AGG TTT GAT TAG G

An den N-terminalen Primer wurde eine Nde I Schnittstelle und an den C-terminalen Primer eine Xho I Schnittstelle angehängt, wie in der Sequenz fett hervorgehoben.

Die Primer wurden nach Verdünnung auf 100 pmol/µl mit der T4-Polynucleotidkinase von Roche phosphoryliert.

### Gewinnung genomische DNA und PCR

Von frisch fermentierter Biomasse des Organismus *Thermoanaerobacter species* wurden 100 mg abgenommen. Die genomische DNA wurde mit den *genomic Tips* der Firma QIAGEN isoliert.

Die PCR-Reaktion wurde mit einem Programm durchgeführt, das einen Annealing-Gradienten von 10°C um die Kerntemperatur von 55°C enthält.

Die restlichen Parameter wurden wie im Roche-high-fidelity-Prototkoll für Fragmente von ca. 1000 Bp beschrieben gewählt.

Die anschließende Gelelektrophorese zeigte in allen 12 Ansätzen ein PCR-Produkt der erwarteten Größe von ca. 1000 Bp.

### Klonierung in pUC 18

Die DNA wurde mit Hilfe der T4-Ligase von Roche in den dem Sma I aufgeschnittenen und dephosphorylierten Vector pUC 18 ligiert. Nach Transformation des Konstruktes in DH5α *E. coli,* Plasmidisolation und Restriktionsanalyse konnten mehrere Klone mit einem Insert tragenden Vektor identifiziert werden.

Das Konstrukt wurde pUC18[ADH T] genannt. Seine Sequenz wurde bestimmt.

### Klonierung in pET24a

Das Gen für die Alkoholdehydrogenase wurde mit den Restriktionsenzymen Nde I und Xho I aus dem Vektor pUC18[ADH T] herausgeschnitten und in den mit den gleichen Enzymen verdauten Vektor pET24a ligiert.

Eine Dephosphorylierung dieses Vektors hat sich bei Vorversuchen als unnötig erwiesen.

Der Ligationsansatz wurde wiederum in DH5α transformiert.

Nach Plasmidisolation und Restriktionsanalyse konnten auch hier mehrere Klone mit dem korrekte Insert isoliert werden.

Das entstandene Konstrukt wurde pET24a[ADH T] genannt und in den Expressions-Host-Stamm Rosetta(DE3)pLacl der Firma Novagen transformiert.

### Expressions-Test

Der so erhaltene Stamm pET24a[ADH T] in Rosetta[DE3]pLacl wurde in LB-Medium für fünf Stunden angezogen, mit 0.5 mM IPTG induziert und über Nacht weiter kultiviert.

Die Zellen wurden geerntet, mit Ultraschall in 100 mM Tris-Cl pH 7.5 aufgeschlossen und die Zelltrümmer abzentrifugiert.

Die Aktivität des überproduzierten Proteins wurde mit dem Substrat Aceton mit 5000 U/g Zellfeuchtgewicht festgestellt.

### Sequenzanalyse

Die festgestellte DNA-Sequenz wurde mit Hilfe des Programms "Clone" in eine Aminosäuresequenz übersetzt und mit der entsprechenden Sequenz aus *Th. ethanolicus* verglichen.

### Hochzelldichtefermentation von E. coli

### Mineralsalzmedium:

| | |
|---|---|
| 4,0 g/L | NaH₂PO₄ x 2 H₂O |
| 14,6 g/L | K₂HPO₄ |
| 0,5 g/L | NH₄Cl |
| 2,5 g/L | (NH₄)₂SO₄ |
| 1,0 g/L | (NH₄)₂H-Citrat |
| 2,0 g/L | Na₂SO₄ |

Das Medium wurde hitzesterilisiert und je 2 mUL 1 M MgSO₄ und Spurenelementlösung aus

| | |
|---|---|
| 0,74 g/L | CaCl₂ x 6 H₂O |
| 0,18 g/L | ZnSO₄ x 7 H₂O |
| 0,1 g/L | MnSO₄ x H₂O |
| 20,1 g/L | Na₂-EDTA |
| 16,7 g/L | FeCl₃ x 6 H₂O |
| 0,1 g/L | CuSO₄ |
| 0,104 g/L | CoCl₂ |

sowie 34 mg/L Thiamin zugesetzt. Als Kohlenstoffquelle dienten 20 g/L Glucose.

Zur Fermentation eines zur Überproduktion der ADH T befähigten Bakteriums, in diesem Fall *E*. *coli,* wurde nun noch Zn₂SO₄ in Konzentrationen zwischen 0 mM und 3 mM zugesetzt.

Das Medium wurde angeimpft und die Sauerstoffsättigung durch geeignete Regulation der Rührerdrehzahl und Begasungsrate auf über 30% gehalten. Nach Verbrauchen der Kohlenstoffquelle wurde Glucose aus einem Feedgefäß nachdosiert, so dass die Sauerstoffkonzentration 30% nicht überstieg.

Der Feed bestand aus dem oben genannten Mineralmedium mit 2 mL/L 1 M MgSO₄, 2 mUL Spurenelementlösung, 34 mg/L Thiamin und 660 g/L Glucose (M.I. Viitanen, A. Vasala, P. Neubauer and T. Alatossava, Microbial Cell Factories 2003, 2:2).

### Enzympräparation:

Die gewonnene Zellmasse wurde in 50 mM Tris-Cl pH 7.0, 1 M NaCl 10 %ig resuspendiert und im Homogenisator aufgeschlossen. Anschließend erfolgte ein Hitzedenaturierungsschritt im Wasserbad für 7.5 min bei 70°C. Die denaturierten Proteine und Zelltrümmer wurden abzentrifugiert.

Der Überstand wurde auf eine Aktivitätskonzentration von 1000 U/mL aufkonzentriert, auf 50 %ige Glycerinkonzentration gebracht und bei -20°C gelagert.

Zur Isolierung der Alkoholdehydrogenase werden bei dem erfindungsgemäßen Verfahren die Wirtszellen vorteilhaft in einem Medium, das Ionen, die ausgewählt sind aus der Gruppe bestehend aus Ionen der Elemente der ersten und zweiten Hauptgruppe des Periodensystems, Ammoniumionen und Eisenionen, in einer Konzentration von wenigstens 0,2 M enthält, homogenisiert und/oder hitzedenaturiert.

Die Erfindung betrifft auch die Verwendung der Alkoholdehydrogenase ADH T zur stereoselektiven Gewinnung von Hydroxyverbindungen durch enzymatische Reduktion von Ketoverbindungen mittels der Alkoholdehydrogenase in einem Medium, das Ionen, die ausgewählt sind aus der Gruppe bestehend aus Ionen der Elemente der ersten und zweiten Hauptgruppe des Periodensystems, Ammoniumionen und Eisenionen, in einer Konzentration von wenigstens 0,2 M enthält.

Die Erfindung betrifft weiterhin ein Verfahren zur stereoselektiven Herstellung von sekundären Alkoholen, bei dem Ketoverbindungen durch eine Alkoholdehydrogenase ADH T in einem Medium, das Ionen, die ausgewählt sind aus der Gruppe bestehend aus Ionen der Elemente der ersten und zweiten Hauptgruppe des Periodensystems, Ammoniumionen und Eisenionen, in einer Konzentration von wenigstens 0,2 M enthält, enzymatisch reduziert werden.

Vorteilhafte Ausgestaltungen des Verfahrens ergeben sich aus den Ansprüchen 11 und 12.

### Sequence

--------
   <213> OrganismName : Thermoanaerobacter species
   <400> PreSequenceString :
<212> Type : DNA
   <211> Length : 1059
   SequenceName : ADH T Nukleotidsequenz
   SequenceDescription :
Feature
   -------
   Sequence: ADH T Nukleotidsequenz:
   <221> FeatureKey : CDS
   <222> LocationFrom : 1
   <222> LocationTo : 1059
   Other Information :
   CDSJoin : No
Custom Codon
   ------------
   Sequence Name : ADH T Nukleotidsequenz

### SEQUENCE LISTING

<110> JFC Juelich Fine Chemicals
   JFC Juelich Fine Chemicals
<120> Expression und Darstellung einer sekundären Alkoholdehydrogenase (ADH-TS) aus Thermoanaerobium bzw. Thermoanaerobacter species zur stereoselektiven Gewinnung von Hydroxyverbindungen
<130> TS-Seq-1
<160> 2
<170> PatentIn version 3.1
<210> 1
   <211> 1059
   <212> DNA
   <213> Thermoanaerobacter species
<220>
   <221> CDS
   <222> (1)..(1059)
   <223>
<400> 1
   atg aaa ggt ttt gca atg ctc agt atc ggt aag gtt ggc tgg att gag      48
   Met Lys Gly Phe Ala Met Leu Ser Ile Gly Lys Val Gly Trp Ile Glu
   1               5                   10                  15
   gta gaa aag cct aat cca gga ccc ttt gat gct atc gta aga ccc cta      96
   Val Glu Lys Pro Asn Pro Gly Pro Phe Asp Ala Ile Val Arg Pro Leu
               20                  25                  30
   gct gtg gcc cct tgc tct tcg gac att cac act gtt ttt gaa gga ggc     144
   Ala Val Ala Pro Cys Ser Ser Asp Ile His Thr Val Phe Glu Gly Gly
           35                  40                  45
   ctt ggt gaa ctt cac aac gca gtg cta ggt cac gaa gct gta ggt gaa     192
   Leu Gly Glu Leu His Asn Ala Val Leu Gly His Glu Ala Val Gly Glu
       50                  55                  60
   gta gtc gaa gtc ggt agt gaa gta aaa gac ttt aaa cct ggt gat aag     240
   Val Val Glu Val Gly Ser Glu Val Lys Asp Phe Lys Pro Gly Asp Lys
   65                  70                  75                  80
   gtg gtc att cct gct atc act cct gat tgg aga acg tta gat gtt caa     288
   Val Val Ile Pro Ala Ile Thr Pro Asp Trp Arg Thr Leu Asp Val Gln
                   85                  90                  95
   cgt ggt tat cat cag cag tcc gga ggt atg ctt gct ggt tac aag ttc     336
   Arg Gly Tyr His Gln Gln Ser Gly Gly Met Leu Ala Gly Tyr Lys Phe
               100                 105                 110
   aca gcc cag aaa cct ggt gtg ttc gcc gag tac atc tac gtt aac gat     384
   Thr Ala Gln Lys Pro Gly Val Phe Ala Glu Tyr Ile Tyr Val Asn Asp
           115                 120                 125
   gca gac atg aat ctt gct cat tta cct gac ggc atc tct tta gaa gcg      432
   Ala Asp Met Asn Leu Ala His Leu Pro Asp Gly Ile Ser Leu Glu Ala
       130                 135                 140
   gcc gtc atg atc aca gat atg atg act acc ggt ttt cac gga gcc gaa      480
   Ala Val Met Ile Thr Asp Met Met Thr Thr Gly Phe His Gly Ala Glu
   145                 150                 155                 160
   ctg gca gaa ata gaa tta ggt gca aca gta gcg gtt ttg ggt att ggt      528
   Leu Ala Glu Ile Glu Leu Gly Ala Thr Val Ala Val Leu Gly Ile Gly
                   165                 170                 175
   cca gta ggt ctt atg gca gtc gct ggt gcc aaa ttg cgg ggt gct gga      576
   Pro Val Gly Leu Met Ala Val Ala Gly Ala Lys Leu Arg Gly Ala Gly
               180                 185                 190
   aga att att gca gta ggc agt aga cct gtt tgt gta gat gct gca aaa      624
   Arg Ile Ile Ala Val Gly Ser Arg Pro Val Cys Val Asp Ala Ala Lys
           195                 200                 205
   tac tat gga gct act gat att gta aac tat aaa aat ggt cct atc gac      672
   Tyr Tyr Gly Ala Thr Asp Ile Val Asn Tyr Lys Asn Gly Pro Ile Asp
       210                 215                 220
   agt cag att atg gat tta acg aaa ggc aaa ggt gtt gat gct gcc atc      720
   Ser Gln Ile Met Asp Leu Thr Lys Gly Lys Gly Val Asp Ala Ala Ile
   225                 230                 235                 240
   atc gct gga gga aat gtt gac atc atg gct aca gca gtt aag att gtt      768
   Ile Ala Gly Gly Asn Val Asp Ile Met Ala Thr Ala Val Lys Ile Val
                   245                 250                 255
   aaa cct ggt ggc acc att gct aat gta aat tac ttt ggc gaa gga gat      816
   Lys Pro Gly Gly Thr Ile Ala Asn Val Asn Tyr Phe Gly Glu Gly Asp
               260                 265                 270
   gtt ttg cct gtt cct cgt ctt gaa tgg ggt tgc ggc atg gct cat aaa      864
   Val Leu Pro Val Pro Arg Leu Glu Trp Gly Cys Gly Met Ala His Lys
           275                 280                 285
   gct ata aaa ggc ggt tta tgc cct ggt gga cgt cta aga atg gaa aga      912
   Ala Ile Lys Gly Gly Leu Cys Pro Gly Gly Arg Leu Arg Met Glu Arg
       290                 295                 300
   ctg att gac ctt gtt ttt tat aag cgt gtc gat cct tcc aaa ctc gtc      960
   Leu Ile Asp Leu Val Phe Tyr Lys Arg Val Asp Pro Ser Lys Leu Val
   305                 310                 315                 320
   act cat gtt ttt caa gga ttt gat aat att gaa aaa gct cta atg ctg     1008
   Thr His Val Phe Gln Gly Phe Asp Asn Ile Glu Lys Ala Leu Met Leu
                   325                 330                 335
   atg aaa gat aaa cca aag gac cta atc aaa cct gtt gta ata tta gca     1056
   Met Lys Asp Lys Pro Lys Asp Leu Ile Lys Pro Val Val Ile Leu Ala
               340                 345                 350
   taa                                                                 1059
<210> 2
   <211> 352
   <212> PRT
   <213> Thermoanaerobacter species
<400> 2
   Met Lys Gly Phe Ala Met Leu Ser Ile Gly Lys Val Gly Trp Ile Glu
   1               5                   10                  15
   Val Glu Lys Pro Asn Pro Gly Pro Phe Asp Ala Ile Val Arg Pro Leu
               20                  25                  30
   Ala Val Ala Pro Cys Ser Ser Asp Ile His Thr Val Phe Glu Gly Gly
           35                  40                  45
   Leu Gly Glu Leu His Asn Ala Val Leu Gly His Glu Ala Val Gly Glu
       50                  55                  60
   Val Val Glu Val Gly Ser Glu Val Lys Asp Phe Lys Pro Gly Asp Lys
   65                  70                  75                  80
   Val Val Ile Pro Ala Ile Thr Pro Asp Trp Arg Thr Leu Asp Val Gln
                   85                  90                  95
   Arg Gly Tyr His Gln Gln Ser Gly Gly Met Leu Ala Gly Tyr Lys Phe
               100                 105                 110
   Thr Ala Gln Lys Pro Gly Val Phe Ala Glu Tyr Ile Tyr Val Asn Asp
           115                 120                 125
   Ala Asp Met Asn Leu Ala His Leu Pro Asp Gly Ile Ser Leu Glu Ala
       130                 135                 140
   Ala Val Met Ile Thr Asp Met Met Thr Thr Gly Phe His Gly Ala Glu
   145                 150                 155                 160
   Leu Ala Glu Ile Glu Leu Gly Ala Thr Val Ala Val Leu Gly Ile Gly
                   165                 170                 175
   Pro Val Gly Leu Met Ala Val Ala Gly Ala Lys Leu Arg Gly Ala Gly
               180                 185                 190
   Arg Ile Ile Ala Val Gly Ser Arg Pro Val Cys Val Asp Ala Ala Lys
           195                 200                 205
   Tyr Tyr Gly Ala Thr Asp Ile Val Asn Tyr Lys Asn Gly Pro Ile Asp
       210                 215                 220
   Ser Gln Ile Met Asp Leu Thr Lys Gly Lys Gly Val Asp Ala Ala Ile
   225                 230                 235                 240
   Ile Ala Gly Gly Asn Val Asp Ile Met Ala Thr Ala Val Lys Ile Val
                   245                 250                 255
   Lys Pro Gly Gly Thr Ile Ala Asn Val Asn Tyr Phe Gly Glu Gly Asp
               260                 265                 270
   Val Leu Pro Val Pro Arg Leu Glu Trp Gly Cys Gly Met Ala His Lys
           275                 280                 285
   Ala Ile Lys Gly Gly Leu Cys Pro Gly Gly Arg Leu Arg Met Glu Arg
       290                 295                 300
   Leu Ile Asp Leu Val Phe Tyr Lys Arg Val Asp Pro Ser Lys Leu Val
   305                 310                 315                 320
   Thr His Val Phe Gln Gly Phe Asp Asn Ile Glu Lys Ala Leu Met Leu
                   325                 330                 335
   Met Lys Asp Lys Pro Lys Asp Leu Ile Lys Pro Val Val Ile Leu Ala
               340                 345                 350

## Patentansprüche

1. Ein DNA-Molekül, das für eine NADP-abhängig (S)-selektive Alkoholdehydrogenase kodiert, wobei die kodierte Alkoholdehydrogenase im Vergleich zu der Alkoholdehydrogenase aus *Thermoanaerobacter ethanolis 39E* durch einen höheren Umsatz bei der Reduktion eines Diketon-Substrats **gekennzeichnet** ist und
(a) eine DNA-Sequenz gemäß SEQ ID NO 1 oder den komplementären Strang derselben aufweist, oder
(b) DNA-Sequenzen aufweist, die mit vorstehenden Sequenzen (a) oder Fragmenten derselben unter stringenten Bedingungen hybridisieren oder
(c) DNA-Sequenzen aufweist, die infolge des genetischen Codes zu DNA-Sequenzen gemäß (a) und (b) degeneriert sind und die für die Alkoholdehydrogenase kodieren.

2. Vektor, **dadurch gekennzeichnet, dass** er mindestens eine Kopie einer DNA-Sequenz nach Anspruch 1 enthält.

3. Prokaryontische oder eukaryontische Wirtszelle, die mit einer DNA-Sequenz nach Anspruch 1 in einer Weise transformiert oder transfiziert ist, die es der Wirtszelle erlaubt, die genannte Alkoholdehydrogenase zu exprimieren.

4. Wirtszelle nach Anspruch 3, **dadurch gekennzeichnet, dass** der Wirt ausgewählt ist aus der Gruppe bestehend aus *Escherichia coli, Saccharomyces cerevisiae, Pichia pastoris und Bacillus subtilis.*

5. Alkoholdehydrogenase, die durch eine der in Anspruch 1 angegebenen DNA-Sequenzen kodiert wird, auch als Teil von Fusionsproteinen.

6. Alkoholdehydrogenase nach Anspruch 5, **dadurch gekennzeichnet, dass** sie eine Aminosäuresequenz gemäß SEQ ID NO 2 aufweist.

7. Verfahren zur Herstellung einer Alkoholdehydrogenase nach Anspruch 5, **dadurch gekennzeichnet, dass** prokaryontische oder eukaryontische Wirtszellen, die mit einer DNA-Sequenz nach Anspruch 1 derart transformiert oder transfiziert worden sind, dass es den Wirtszellen ermöglicht ist, die Alkoholdehydrogenase zu exprimieren, in einem Kultivierungsmedium, das Zinkionen in einer Konzentration von 0,1 bis 5 mM enthält, kultiviert werden und die Alkoholdehydrogenase als Expressionsprodukt der DNA-Sequenz isoliert wird.

8. Verfahren nach Anspruch 7, **dadurch gekennzeichnet, dass** zur Isolierung der Alkoholdehydrogenase die Wirtszellen in einem Medium, das Ionen, die ausgewählt sind aus der Gruppe bestehend aus Ionen der Elemente der ersten und zweiten Hauptgruppe des Periodensystems, Ammoniumionen und Eisenionen, in einer Konzentration von wenigstens 0,2 M enthält, homogenisiert und/oder hitzedenaturiert werden.

9. Verwendung der Alkoholdehydrogenase nach Anspruch 5 oder 6 zur stereoselektiven Gewinnung von Hydroxyverbindungen durch enzymatische Reduktion von Ketoverbindungen mittels der Alkoholdehydrogenase in einem Medium, das Ionen, die ausgewählt sind aus der Gruppe bestehend aus Ionen der Elemente der ersten und zweiten Hauptgruppe des Periodensystems, Ammoniumionen und Eisenionen, in einer Konzentration von wenigstens 0,2 M enthält.

10. Verfahren zur stereoselektiven Herstellung von sekundären Alkoholen, **dadurch gekennzeichnet, dass** Ketoverbindungen durch eine Alkoholdehydrogenase nach Anspruch 5 oder 6 in einem Medium, das Ionen, die ausgewählt sind aus der Gruppe bestehend aus Ionen der Elemente der ersten und zweiten Hauptgruppe des Periodensystems, Ammoniumionen und Eisenionen, in einer Konzentration von wenigstens 0,2 M enthält, enzymatisch reduziert werden.

11. Verfahren nach Anspruch 10, **dadurch gekennzeichnet, dass** die Ketoverbindung ausgewählt ist aus der Gruppe bestehend aus aliphatischen und aromatischen Ketonen, 2- oder 3- Ketoestern und Diketonen.

12. Verfahren nach Anspruch 10, **dadurch gekennzeichnet, dass** die Ketoverbindung ausgewählt ist aus der Gruppe bestehend aus 2-Butanon, 2-Hexanon, 2-Oktanon, Monochloraceton, Acetaldehyd, 2-Methylbutylraldehyd, Diacetyl, 2,4-Pentandion, 2,5-Hexandion, Ethylacetoacetat, Ethyl 4-Chloracetoacetat, Acetophenon, p-Chloracetophenon, 2-Acetylpyridin, 3-Butyn-2-on, 1,2-Cyclohexandion, Pyruvat, 2-Oxobuttersäure, 4-Chlor-2-butanon, 4-Hydroxy-2-butanon, 3-Oktanon und Phenacylchlorid.

## Claims

1. DNA molecule coding for an NADP-dependent (S)-selective alcohol dehydrogenase, wherein the coded alcohol dehydrogenase is **characterised by** a higher conversion rate in the reduction of a diketone substrate compared with the alcohol dehydrogenase from *Thermoanaerobacter ethanolis 39E* and
(a) has a DNA sequence according to SEQ ID NO 1 or the complementary strand thereof,
(b) has DNA sequences that hybridize with the sequences (a) above or with fragments thereof under stringent conditions or
(c) has DNA sequences that are degenerated due to the genetic code into DNA sequences according to (a) and (b) and that code for the alcohol dehydrogenase.

2. Vector, **characterized in that** it contains at least one copy of a DNA sequence according to claim 1.

3. Prokaryotic or eukaryotic host cell that is transformed or transfected with a DNA sequence according to claim 1 in such a way that the host cell is allowed to express said alcohol dehydrogenase.

4. A host cell according to claim 3, **characterised in that** the host is selected from the group consisting of *Escherichia coli, Saccharomyces cerevisiae, Pichia pastoris* and *Bacillus subtilis.*

5. Alcohol dehydrogenase that is coded by one of the DNA sequences given in claim 1, also as a part of fusion proteins.

6. Alcohol dehydrogenase according to claim 5, **characterised in that** it contains an amino acid sequence according to SEQ ID NO 2.

7. Method for the production of an alcohol dehydrogenase according to claim 5, **characterised in that** prokaryotic or eukaryotic host cells which are transformed or transfected with a DNA sequence according to claim 1 in such a way that the host cells are enabled to express said alcohol dehydrogenase are cultivated in a culture medium containing zinc ions in a concentration of 0.1 to 5 mM, and the alcohol dehydrogenase is isolated as expression product of the DNA sequence.

8. Method according to claim 7, **characterised in that** the host cells are homogenised and/or heat-denatured in a medium containing ions that are selected from the group consisting of ions of the elements of the first and second main groups of the periodic table, ammonium ions and iron ions, in a concentration of at least 0.2 M.

9. Use of the alcohol dehydrogenase according to claim 5 or 6 for the stereoselective production of hydroxy compounds by enzymatic reduction of keto compounds by means of the alcohol dehydrogenase in a medium containing ions that are selected from the group consisting of ions of the elements of the first and second main groups of the periodic table, ammonium ions and iron ions, in a concentration of at least 0.2 M.

10. Method for the stereoselective production of secondary alcohols, **characterised in that** keto compounds are enzymatically reduced by an alcohol dehydrogenase according to claim 5 or 6 in a medium containing ions that are selected from the group consisting of ions of the elements of the first and second main groups of the periodic table, ammonium ions and iron ions, in a concentration of at least 0.2 M.

11. Method according to claim 10, **characterised in that** the keto compound is selected from the group consisting of aliphatic and aromatic ketones, 2- or 3-ketoesters and diketones.

12. Method according to claim 10, **characterised in that** the keto compound is selected from the group consisting of 2-butanone, 2-hexanone, 2-octanone, monochloroacetone, acetaldehyde, 2-methyl butyraldehyde, diacetyl, 2,4-pentanedione, 2,5-hexanedione, ethyl acetoacetate, ethyl-4-chloroacetoacetate, acetophenone, p-chloroacetophenone, 2-acetylpyridine, 3-butyn-2-one, 1,2-cyclohexanedione, pyruvate, 2-oxobutanoic acid, 4-chlor-2-butanone, 4-hydroxy-2-butanone, 3-octanone and phenacyl chloride.

## Revendications

1. Molécule d'ADN codée pour un alcool déshydrogénase (S)-sélectif NADP-dépendant, dans laquelle l'alcool déshydrogénase codé est **caractérisé par** un taux plus élevé lors de la réduction du substrat de dicétone comparé à l'alcool déshydrogénase issu de *Thermoanaerobacter ethanolis 39E,* et
(a) présente une séquence d'ADN selon SEQ ID NO 1 ou le brin complémentaire de celle-ci, ou
(b) présente des séquences d'ADN qui s'hybrident avec des séquences existantes (a) ou des fragments de celles-ci dans des conditions stringentes ou
(c) présente des séquences d'ADN qui dégénèrent suivant le code génétique des séquences d'ADN selon (a) et (b) et qui codent pour l'alcool déshydrogénase.

2. Vecteur **caractérisé en ce qu'**il contient au moins une copie d'une séquence d'ADN selon la revendication 1.

3. Cellule hôte procaryote ou eucaryote qui est transformée ou transfectée avec une séquence d'ADN selon la revendication 1 de sorte à permettre à la cellule hôte d'exprimer ledit alcool déshydrogénase.

4. Cellule hôte selon la revendication 3, **caractérisée en ce que** l'hôte est sélectionné dans le groupe constitué *d'Escherichia coli,* de *Saccharomyces cerevisiae,* de *Pichia pastoris* et de *Bacillus subtilis.*

5. Alcool déshydrogénase qui est codé par l'une des séquences d'ADN indiquées dans la revendication 1, également en tant que partie de protéines de fusion.

6. Alcool déshydrogénase selon la revendication 5, **caractérisé en ce qu'**il présente une séquence d'acides aminés selon SEQ ID NO 2.

7. Procédé de fabrication d'un alcool déshydrogénase selon la revendication 5, **caractérisé en ce que** des cellules hôtes procaryotes ou eucaryotes qui ont été transformées ou transfectées avec une séquence d'ADN selon la revendication 1 de sorte à permettre aux cellules hôtes d'exprimer l'alcool déshydrogénase, dans un milieu de culture qui contient des ions zinc dans une concentration comprise entre 0,1 et 5 mM, sont cultivées et l'alcool déshydrogénase est isolé en tant que produit d'expression de la séquence d'ADN.

8. Procédé selon la revendication 7, **caractérisé en ce que**, pour isoler l'alcool déshydrogénase, les cellules hôtes sont homogénéisées et/ou dénaturées par la chaleur dans un milieu qui contient des ions sélectionnés dans le groupe constitué d'ions d'éléments des premier et second groupes principaux du système périodique, d'ions d'ammonium et d'ions ferreux, dans une concentration d'au moins 0,2 M.

9. Utilisation de l'alcool déshydrogénase selon la revendication 5 ou 6 pour l'obtention stéréosélective de liaisons hydroxyle par réduction enzymatique de liaisons cétoniques au moyen de l'alcool déshydrogénase dans un milieu qui contient des ions sélectionnés dans le groupe constitué d'ions d'éléments des premier et second groupes principaux du système périodique, d'ions d'ammonium et d'ions ferreux, dans une concentration d'au moins 0,2 M.

10. Procédé de fabrication stéréosélective d'alcools secondaires, **caractérisé en ce que** des liaisons cétoniques sont réduites de façon enzymatique par un alcool déshydrogénase selon la revendication 5 ou 6 dans un milieu qui contient des ions sélectionnés dans le groupe constitué d'ions d'éléments des premier et second groupes principaux du système périodique, d'ions d'ammonium et d'ions ferreux, dans une concentration d'au moins 0,2 M.

11. Procédé selon la revendication 10, **caractérisé en ce que** la liaison cétonique est sélectionnée dans le groupe constitué de cétones aliphatiques et aromatiques, de 2- ou 3-cétoesters et de dicétones.

12. Procédé selon la revendication 10, **caractérisé en ce que** la liaison cétonique est sélectionnée dans le groupe constitué de 2-butanone, 2-hexanone, 2-octanone, monochloracétone, acétaldéhyde, 2-méthylbutyraldéhyde, diacétyle, 2,4-pentanedione, 2,5-hexanedione, éthylacétoacétate, éthyl 4-chloracétoacétate, acétophénone, p-chloracétophénone, 2-acétylpyridine, 3-butyn-2-one, 1,2-cyclohexanedione, pyruvate, 2-oxoacide butyrique, 4-chlor-2-butanone, 4-hydroxy-2-butanone, 3-octanone et phénacylchlorure.
